**Europäisches Patentamt**

**European Patent Office**

**Office européen des brevets**

(11) Numéro de publication : **0 375 520 B1**

(12)

# FASCICULE DE BREVET EUROPEEN

(45) Date de publication du fascicule du brevet :
07.04.93 Bulletin 93/14

(51) Int. Cl.⁵ : **A61K 9/16,** A61K 7/00

(21) Numéro de dépôt : **89403488.3**

(22) Date de dépôt : **14.12.89**

(54) Composition cosmétique ou pharmaceutique contenant des microsphères de polymères ou de corps gras chargées d'au moins un produit actif.

(30) Priorité : **20.12.88 LU 87410**

(43) Date de publication de la demande :
**27.06.90 Bulletin 90/26**

(45) Mention de la délivrance du brevet :
**07.04.93 Bulletin 93/14**

(84) Etats contractants désignés :
**AT BE CH DE ES FR GB GR IT LI NL SE**

(56) Documents cités :
EP-A- 0 211 298
EP-A- 0 316 054
FR-A- 2 218 086
FR-A- 2 530 250
P. BURI et al.: "Formes Pharmaceutiques Nouvelles", 1985, Technique et Documentation (Lavoisier), Paris, FR

(73) Titulaire : **CENTRE INTERNATIONAL DE RECHERCHES DERMATOLOGIQUES GALDERMA - CIRD GALDERMA**
**Sophia Antipolis**
**F-06560 Valbonne (FR)**

(72) Inventeur : **Schaefer, Hans**
**Villa La Bergerie Chemin de la Constance**
**F-06600 Antibes (FR)**
Inventeur : **Watts, Francine**
**33, Rue des Abris**
**F-06620 Le Bar sur Loup (FR)**
Inventeur : **Papantoniou, Christos**
**17, Rue des Basserons**
**F-95160 Montmorency (FR)**
Inventeur : **Mahieu, Claude**
**90, Avenue de Villiers**
**F-75017 Paris (FR)**

(74) Mandataire : **Peuscet, Jacques et al**
**Cabinet Peuscet 68, rue d'Hauteville**
**F-75010 Paris (FR)**

EP 0 375 520 B1

## Description

La présente invention concerne une composition cosmétique ou pharmaceutique contenant des microsphères de polymères ou de corps gras chargées d'au moins un produit actif dans un véhicule approprié.

Il est connu, dans l'état de la technique, de préparer des microcapsules dans lesquelles le principe actif est enfermé et n'est pas en contact avec le milieu extérieur (voir notamment le brevet français 2 218 086 et le brevet européen 316 054). Cependant, au moment de l'application, la micro-capsule peut se briser prématurément et libérer immédiatement le principe actif.

Il est également connu de préparer des polymères naturels ou synthétiques sous forme de microsphères par réticulation de ces polymères en suspension. Un procédé de fabrication de microsphères de poly-$\beta$-alanine est, par exemple, décrit dans le brevet français 2530250. Il est aussi connu de préparer des microsphères de corps gras.

On sait aussi que ces microsphères sont susceptibles de se charger en produits chimiques, en particulier en produits actifs (voir notamment le brevet français précité et le brevet des Etats-Unis d'Amérique 4690825). Dans la présente demande, on entend par produit actif tout produit ayant une activité du point de vue cosmétique ou pharmaceutique. Le produit solide constituant la miscrosphère peut, en effet, servir de support absorbant ou adsorbant, ou encore, de liant pour de nombreux produits chimiques (voir le brevet européen 211 298). Les microsphères chargées en produits actifs sont utilisées dans un véhicule approprié dans lequel le support solide constituant les microsphères est très peu ou pas du tout soluble. Ce véhicule peut être une solution aqueuse ou une phase huileuse.

Une composition cosmétique ou pharmaceutique contenant de telles microsphères chargées de produit(s) actif(s) dans un véhicule approprié est utilisable pour amener des médicaments en un point déterminé du corps, en particulier par application sur la peau. Cependant, l'application topique n'a généralement pas l'efficacité désirée car l'épiderme forme barrière.

Selon la présente invention, on a trouvé que, si les microsphères de la composition cosmétique ou pharmaceutique sont choisies dans une gamme de dimensions particulière, l'efficacité du produit actif qu'elles contiennent est, de façon très inattendue, fortement augmentée. Les études menées par la société déposante ont permis de constater que cette amélioration considérable était liée a la pénétration des microsphères dans les follicules sébacés.

La présente invention a donc pour objet une composition cosmétique ou pharmaceutique pour application topique contenant, dans un véhicule approprié, des microsphères de polymères naturels ou synthétiques ou de corps gras de point de fusion supérieur à 50°C chargées d'au moins un produit actif, caractérisée par le fait qu'au moins 80 % en poids des microsphères utilisées ont un diamètre compris entre 3 $\mu$m et 10 $\mu$m.

En effet, les microsphères ayant un diamètre dans la gamme définie ci-dessus, pénètrent dans le follicule sébacé mais peu dans la peau. Lesdites microsphères atteignent donc sélectivement et progressivement le canal folliculaire où le produit actif, qu'elles portent, diffuse dans le canal folliculaire et les tissus environnants. Par contre, le support, qui constitue la microsphère, est ensuite rejeté grâce à l'écoulement du sébum et/ou à la pousse du poil. On évite ainsi toute réaction indésirable de l'organisme vis-a-vis du composé solide constituant les microsphères.

Il faut noter que, lorsque les microsphères ont un diamètre inférieur à 3 $\mu$m, elles pénètrent aussi dans les canaux folliculaires mais également dans la couche cornée, en concentration élevée. Or, ce relargage du principe actif dans la couche cornée, dans le cas de préparations anti-acnéiques par exemple, se traduit par l'apparition d'effets secondaires indésirables dans la mesure où le produit actif est libéré dans les zones de peau saine touchées par l'application, qui entourent les canaux folliculaires; tandis que, dans le cas de médicaments à action par voie systémique, le produit actif est libéré dans une zone non vascularisée où, de plus, intervient la barrière cornée. Le relargage du principe actif dans la couche cornée correspond donc globalement, dans les deux cas, à une réduction d'efficacité de la composition. Lorsque les microsphères ont un diamètre supérieur à environ 10$\mu$m, elles restent pour la plupart localisées sur la surface de la peau sans y pénétrer, d'où une inefficacité de l'application topique puisque le produit actif ne peut être relargué que sur la couche cornée. Dans les deux cas, le ciblage des produits actifs est nettement inférieur à celui que l'on obtient en mettant en oeuvre l'invention.

En d'autres termes, l'invention propose de sélectionner la dimension des microsphères de façon à favoriser leur pénétration sélective dans les follicules sébacés; dans le cas de l'acné, le produit actif est ainsi amené spécifiquement sur les zones-cibles sans effets secondaires indésirables sur les zones de peau saine entourant les canaux folliculaires; dans le cas où le produit actif est un médicament ayant une action par voie systémique, le canal folliculaire constitue une voie d'administration générale très efficace dans la mesure où la diffusion du produit actif dans ce compartiment débouche sur une région très vascularisée.

Il n'était pas évident que les microsphères susceptibles de pénétrer dans le follicule pileux devaient avoir les dimensions définies ci-dessus. En effet, le diamètre moyen des orifices pilo-sébacés est compris dans une gamme dimensionnelle tout à fait différente de celle indiquée ci-dessus pour les microsphères par exemple, sur le front, ce diamètre moyen est compris entre 52 μm et 82 μm. Chez l'homme, la surface des orifices pilo-sébacés situés sur le front est d'environ Ø,ØØ2 mm2 (W.J. Cunliffe, W.D.H. Perera, P. Thackray, M. Williams, R.A. Forster and S.M. Williams, British Journal of Dermatology, 1976, 95, 153). En supposant que le contour du canal folliculaire est à peu près circulaire, on peut estimer, d'après cet article, le diamètre moyen des orifices pilo-sébacés à 5Ø,5 μm. Celui-ci, redéterminé par la demanderesse par mesure de la taille des orifices pilo-sébacés situés sur la peau du front de six volontaires sains, est trouvé compris entre 52 μm et 82 μm (voir étude décrite dans l'essai B de la présente demande). Cette différence considérable entre la gamme des diamètres des orifices pilo-sébacés et la gamme des diamètres des microsphères efficaces rendait l'invention particulièrement surprenante pour l'homme de métier. Ce caractère surprenant est d'ailleurs confirmé par le fait que, dans le brevet des Etats-Unis d'Amérique 469Ø825 précité, les indications dimensionnel les fournies ne visent que des microsphères ayant des diamètres compris entre 1Ø et 1ØØ μm.

On peut sélectionner les microsphères ayant la dimension désirée par tamisage, notamment en milieu humide, des microsphères obtenues par un procédé donnant des microsphères ayant une gamme de dimensions plus étendue. On peut également obtenir des microsphères dont les dimensions sont contenues dans la gamme désirée en orientant convenablement le procédé de fabrication des microsphères. On peut, par exemple, ajuster la taille des microsphères en choisissant le solvant de polymérisation, l'agent de réticulation, ou en modifiant la vitesse et le temps d'agitation du milieu réactionnel. Ces différentes modifications font partie de l'état de la technique et/ou sont du domaine de l'homme de l'art.

Les polymères naturels ou synthétiques utilisables pour la fabrication des microsphères de la composition de la présente invention, sont choisis parmi ceux susceptibles d'être appliqués sur la peau sans effet indésirable et susceptibles de former des microsphères ayant les dimensions désirées. Ils doivent également être compatibles avec le produit actif utilisé.

Les polymères utilisables dans les compositions de la présente invention, peuvent avantageusement être choisis parmi :
- les polymères à base de styrène, tels que le polystyrène ;
- les polymères à base de β-alanine, tels que la poly-β-alanine ;
- les polymères dérivés de l'acide acrylique ou méthacrylique ;
- les polyesters dérivés de l'acide lactique et/ou glycolique ;
- les protéines réticulées :
  . soit par le glutaraldéhyde ou par un dichlorure d'acide tel que le chlorure de térephtaloyle,
  . soit en présence d'un activateur tel qu'un carbodiimide ;
- les protéines coagulées par la chaleur (albumine).

De préférence, les polymères utilisables sont choisis parmi les polymères à base de poly-β-alanine et les polyesters dérivés de l'acide lactique ou glycolique.

Les corps gras utilisables peuvent être choisis parmi:
- les dérivés d'alcools et d'acides gras, tels que la tristéarine, les triglycérides semi-synthétiques ou le monostéarate de glycérol ;
- les alcools gras tel que l'alcool cétylique.

De préférence, les corps gras utilisables sont choisis parmi les corps gras ayant un point de fusion compris entre 50°C et 100°C environ.

Les produits actifs utilisables dans la composition selon l'invention sont ceux susceptibles d'être appliqués sur la peau. Ils peuvent être choisis parmi :
- les agents de traitement de l'acné tels que les composés à action de type rétinoïde (vitamine A, acide rétinoïque ou ses dérivés) ;
- le peroxyde de benzoyle ;
- les facteurs de croissance de nature peptidique, tels que le facteur de croissance épidermique (EGF) ou protéinique ;
- les agents de raffermissement de la peau tels que le nicotinate de benzyle ;
- les agents de traitement des cheveux, en particulier, les agents anti-chute ou de repousse des cheveux, tels que le minoxidil, et les antiséborrhéiques comme la S-carboxyméthylcystéine ou l'octopirox ;
- les antifongiques, comme la nystatine ou l'econazole ;
- les astringents, tels que le chlorure d'aluminium ;
- les antibiotiques, tels que l'érythromycine et la tétracycline ;
- les anti-viraux, tels que la vidarabine ;
- les antihypertenseurs, tels que le chlorhydrate de clonidine ;
- les anti-angineux, tels que la nitroglycérine ;

- les vasodilatateurs, comme la bradikynine ;
- les agents de traitement des maladies cardiovasculaires, tels que les peptides du groupe des tachykinines, par exemple, la "substance P" ;
- les agents anti-inflammatoires, comme l'aspirine ou l'hydrocortisone et ses dérivés ;
- les anti-allergènes, tels que les chromoglycates ;
- les antiprurigineux, tels que les dérivés de la phénothiazine ;
- les neurostimulants, tels que la caféine ou la théophylline ;
- les agents antidépresseurs, tels que les sels de lithium et, plus particulièrement, le carbonate de lithium ;
- les composés naturels utilisés en recherche neurobiologique, tels que la capsaïcine ;
- les anesthésiants, tels que la lidocaïne et la procaïne.
- les stéroïdes hormonaux tels que le $17\text{-}\alpha\text{-}$oestradiol et le $17\text{-}\beta\text{-}$oestradiol.

Le véhicule approprié est sous forme aqueuse ou sous forme d'huile.

Le véhicule sous forme aqueuse peut être un gel aqueux obtenu à l'aide d'un agent gélifiant, tel que l'acide polyacrylyque réticulé vendu sous la dénomination commerciale "Carbopol" par la société "Goodrich BF", ou les dérivés cellulosiques vendus sous la dénomination commerciale "Klucel" par la société "Hercules" ; ou un gel hydroalcoolique contenant par exemple du propylèneglycol. On peut également utiliser une solution aqueuse lipophile telle qu'une solution aqueuse de silicones.

Les huiles utilisables comme véhicules sont des huiles liquides ou semi-solides tels que les triglycérides d'acides gras en C8-C1∅, et leurs mélanges, la vaseline, l'huile de vaseline, la lanoline.

Le pH du véhicule est, de préférence, ajusté à une valeur basique.

Le véhicule est sous forme de liquide, de gel, de crème, de pâte, de pommade ou de poudre sèche. Pour obtenir une pâte, une pommade ou un onguent, on ajoute un excipient, tel que le polyéthylène-glycol, une cire, telle qu'une cire d'abeille, ou de la lanoline.

De façon générale, les compositions cosmétique ou pharmaceutique selon la présente invention, contiennent de 1 % à 4∅ % en poids de microsphères, dont au moins 8∅ % ont des diamètres compris entre 3 et 1∅ µm.

Elles contiennent également de ∅,∅5 % à 6∅ % en poids de produit actif.

Les microsphères sont fabriquées par tout procédé connu. Les microsphères de polystyrène sont largement commercialisées. Celles de poly-$\beta$-alanine peuvent, par exemple, être préparées selon les procédés décrits dans le brevet français 253∅25∅.

Pour introduire le produit actif dans la microsphère, on dissout le produit actif dans un solvant ou un mélange de solvants ayant une affinité suffisante vis-à-vis du composé constituant les microsphères. Parmi les solvants appropriés, spécialement pour les sphères de poly-$\beta$-alanine, on peut citer, par exemple, l'eau, le glycérol, l'éthanol, le diéthylène-glycol, l'acétone et en général, les solvants organiques miscibles à l'eau.

Lorsque l'on a utilisé un solvant pour obtenir les microsphères chargées de produit actif, on peut utiliser lesdites microsphères telles quelles ou après élimination du solvant qui y subsiste. Ce solvant peut y avoir subsisté comme solvant du produit actif et/ou comme agent de gonflement de la microsphère elle-même lorsque le polymère, dont elle est constituée, est susceptible de gonfler dans ledit solvant. Lorsque les microsphères sont utilisées après élimination du solvant, le produit actif reste néanmoins, au séchage, piégé dans (ou : sur) la microsphère. Le gonflement du polymère, par un solvant, conduit a des microsphères sous forme de gel, sous réserve que la quantité de solvant ne dépasse pas certaines limites, qui sont différentes selon le polymère constituant les microsphères. Les microsphères chargées d'au moins un produit actif, séchées ou non, sont mélangées au véhicule choisi.

La composition cosmétique ou pharmaceutique obtenue est appliquée de façon usuelle sur la peau, de préférence avec un léger massage. Dans une variante, les microsphères sont chargées d'un produit actif sous forme ionisée : dans ce cas, après application de la composition sur la peau, le relargage du produit actif peut être accéléré par ionophorèse.

Les exemples donnés ci-dessous, à titre purement illustratif et non limitatif, permettront de mieux comprendre l'invention. Les essais A, B et C sont des mesures fournies pour expliquer la remarquable efficacité des compositions selon l'invention.

Essai A :

Dans cet essai, on évalue la taille des orifices pilosébacés chez l'homme. Cette étude a été effectuée chez six volontaires sains (trois hommes et trois femmes) âgés de 25 à 35 ans et elle a été faite sur la peau du front.

Après avoir soigneusement nettoyé au savon une zone de peau d'environ 2 cm2, on choisit un colorant (coloration directe "L'OREAL RENOVATIVE", brun profond, commercialisé par la société dite "l'OREAL") et on l'applique, pendant quinze minutes, sur la partie latérale gauche ou droite du front de chaque sujet. A la fin du temps de pose, la zone colorée est nettoyée avec un peu d'eau, afin d'éliminer l'excès de colorant. Cette zone est photographiée avec un montage macrophotographique réalisé à l'aide d'un appareil Olympus. Cet appareil permet d'effectuer des photographies standardisées de la zone a analyser (même distance et même grandissement pour tous les sujets). Le colorant utilisé n'est plus visible 24 heures après l'application.

La distribution de tailles des orifices pilo-sébacés est réalisée par analyse d'image à l'aide de l'appareil "Quantimet 52Ø" de la Société "Cambridge Instruments", à partir de diapositives du front. L'appareil mesure la surface S des embouchures de follicule et calcule le diamètre D de chaque follicule d'après la formule :

$D = 2 (S/\Pi)$

Les résultats sont donnés dans le tableau I ci-après.

Le diamètre moyen des follicules est trouvé compris entre 52 µm et 82 µm pour l'ensemble des sujets étudiés.

Essai B :

Des essais ont été effectués pour mettre en évidence la relation entre la taille des microsphères et leur pénétration à travers la couche cornée et les follicules de la peau humaine.

Ces essais ont utilisé des microsphères de polystyrène fluorescentes de différents calibres entre 1 µm et 24 µm ayant les caractéristiques données dans le tableau II ci-après. Ces lots de microsphères de polystyrène ont été mis en suspension à 1Ø % en poids dans un mélange de triglycérides d'acides gras en C8-C1Ø commercialisés sous la marque "Mygliol 812" par la société "Dynamit Nobel" ; les tests ont été réalisés sur de la peau de lifting du visage de patientes âgées de 44 à 66 ans.

## TABLEAU I

| SUJET N° | SEXE | FOLLICULES SURFACE | DIAMETRE (µm) | | |
|----------|------|--------------------|----------------|-------|-------|
| | | | Moyenne +/- Ecart-Type | <90% | <95% |
| 1 | M | 105 | 82 +/- 34 | <128 | <150 |
| 2 | F | 102 | 68 +/- 42 | <120 | <141 |
| 3 | F | 111 | 82 +/- 43 | <133 | <158 |
| 4 | F | 116 | 52 +/- 25 | < 87 | < 99 |
| 5 | M | 108 | 79 +/- 37 | <128 | <143 |
| 6 | M | 68 | 79 +/- 31 | <124 | <132 |

## TABLEAU II

| Diamètre moyen* (μm) | | Ecart-type* (μm) | Référence des micro-sphères de POLYSCIENCES INC | Type de** fluo-rescence |
|---|---|---|---|---|
| Valeur exacte | Valeur arrondie | | | |
| 0,91 | 1 | 0,06 | 17 154 | jaune-vert |
| 1,17 | 1 | 0,04 | 17 458 | bleu brillant |
| 3,1 | 3 | 0,1 | 17 155 | jaune-vert |
| 6,83 | 7 | 0,2 | 18 141 | jaune-vert |
| 7,0 | 7 | 0,3 | 17 156 | jaune-vert |
| 9,13 | 9 | 0,6 | 18 140 | jaune-vert |
| 9,55 | 10 | 1,53 | 18 142 | jaune-vert |
| 23,8 | 24 | 4,2 | 18 241 | jaune-vert |

\* Les analyses de tailles de ces étalons de granulométries ont été fournies par OSI (POLYSCIENCES INC.).

** Type de fluorescence : (voir tableau III).

## TABLEAU III

| Fluorescence | Excitation max. (nm) | Emission max. (nm) |
|---|---|---|
| Bleu brillant | 365 | 468 |
| Jaune-vert | 458 | 540 |

Les applications sont réalisées, environ 4 heures après l'excision chirurgicale, sur de la peau de visage non congelée (conservation a 4° C en chambre froide). La peau est débarassée de son tissu sous-cutané à l'aide d'un scalpel, puis légèrement étirée et épinglée sur un support recouvert d'aluminium. La surface cutanée est soigneusement nettoyée par essuyage avec un mouchoir en papier, suivi d'un léger "stripping" réalisé avec du ruban adhésif vendu sous la dénomination commerciale "Transpore". Les différentes suspensions de microbilles sont alors appliquées avec une spatule en verre durant 15 minutes, avec 5 minutes de massage, à l'intérieur de sites d'application de 2,5 cm2 délimités par des rondelles en plastique collées par une colle à base de polymère de cyanoacrylate, commercialisée sous la dénomination de "Cyanolit". A la fin du temps d'application, l'excédent de produit n'ayant pas pénétré dans la peau est enlevé avec un coton-tige suivi de trois applications très légères, à la surface de la peau, d'un morceau de ruban adhésif de dénomination commerciale "Transpore" (adhérant peu à la peau et ne provoquant pas de délaminage de la couche cornée). Des biopsies des sites d'application, ainsi que d'une zone de peau témoin sans application, sont prélevées avec un emporte-pièce "Punch biopsy" de 6 mm de diamètre et congelées dans de l'azote liquide. La pénétration des microbilles dans la couche cornée et les follicules est alors mise en évidence, au microscope optique à fluorescence (Photomicroscope IIIRS, Zeiss, West Germany) sur des coupes de peau verticales en congélation de 1Ø μm à 15 μm d'épaisseur, réalisées à l'aide d'un cryomicrotome (Cryostat Bright, Bright Instrument Company Limited).

Les résultats obtenus sont les suivants :

- les microsphères de 24 μm de diamètre restent localisées sur la surface de la peau sans y pénétrer ;
- les microsphères de 9 μm à 1Ø μm de diamètre ont tendance à se rassembler autour des canaux folliculaires ;

- les microsphères de 7 µm ont pu être localisées de façon sélective à l'intérieur des follicules sébacés ;
- les microsphères de 1 µm à 3 µm ont tendance a pénétrer aussi bien dans la couche cornée que dans les follicules.

Essai C :

Des essais ont été effectués pour mettre en évidence la relation entre la taille des microsphères et leur pénétration dans la couche cornée et les follicules chez le rat.

Ces essais ont utilisé des microsphères de poly-β-alanine on a testé trois échantillons ayant respectivement des diamètres moyens d'environ 2 µm, 5 µm et 12 µm.

Les microsphères utilisées sont préparées par réticulation de la poly-β-alanine à l'aide de glutaraldéhyde. Cette synthèse est décrite dans le brevet français 253Ø25Ø. Ces microsphères sont ensuite rendues fluorescentes par une réaction intermédiaire de l'hexaméthylènediamine sur les fonctions aldéhydiques résiduelles, présentes à leur surface, suivie d'une réaction avec le chlorure de dansyle. Les microsphères obtenues présentent une forte fluorescence verte, très homogène, en lumière ultra-violette. Ces microsphères ont les caractéristiques suivantes :

- échantillon 1 : diamètre = 1,79 ± Ø,86 µm (9Ø% au-dessous de 2,9 µm) ;
- échantillon 2 : diamètre = 4,8 ± 1,1 µm (9Ø % au-dessous de 6,1 µm) préparé selon l'Exemple 1 ;
- échantillon 3 : diamètre 12,4 ± 2,2 µm (9Ø % au-dessous de 15,1 µm).

Ces mesures de tailles ont été déterminées par la technique d'analyse d'image en fluorescence sur un appareil "QUANTIMET 52Ø" commercialisé par la Société "CAMBRIDGE INSTRUMENTS CO".

Le protocole d'application utilisé est le suivant : après anesthésie au pentobarbital (dose 3Ø mg/kg), des sites d'application de 5 cm2 sont délimités par une rondelle en plastique collée sur le dos du rat nu femelle ICO (poids moyen 17Ø-18Øg). Les différentes suspensions sont appliquées durant 2 heures, à raison de 5 à 1Ø mg/cm2, à l'intérieur de ces sites. Afin de tester l'influence du massage sur la pénétration des microsphères de poly-β-alanine dans les follicules sébacés, l'application est réalisée en comparant deux temps de massage : une minute et cinq minutes. L'animal est bandé pendant toute la durée de l'expérience, afin d'éviter tout contact avec la zone d'application. Au bout de 2 heures, l'excédent de produit n'ayant pas pénétré dans la peau est soigneusement enlevé avec un coton-tige ; on réalise ensuite trois applications très légères, à la surface de la peau, d'un morceau de ruban adhésif de dénomination commerciale "Transpore" (adhérant peu à la peau et ne provoquant pas de délaminage de la couche cornée). Des biopsies des zones d'application sont prélevées (6 mm de diamètre) et congelées dans de l'azote liquide. La pénétration des microsphères dans les compartiments cornéen et folliculaire est alors mise en évidence au microscope optique à fluorescence sur des coupes de peau verticales en congélation, de 1Ø µm à 15 µm d'épaisseur, réalisées au cryomicrotome.

Afin de tester l'influence du véhicule sur la pénétration des microsphères de poly-β-alanine, celles-ci sont formulées, à la concentration de 1Ø % en poids, dans les véhicules suivants :

1) Gel aqueux ayant la formulation suivante :

```
Acide polyacrylique réticulé vendu
sous la dénomination commerciale
"Carbopol 94Ø" par la Société
"Goodrich BF"........................  Ø,4g
Soude (solution aqueuse à 1Ø% en poids) 2,Øg
Eau .............................q.s.p 1ØØ,Øg
```

2) Véhicule eau-silicone constitué de :

```
Eau ..............................  5,Øg
Huile de silicone vendue par la
Société "Dow Corning" sous la
référence "Q2-3225c" ..........q.s.p 1ØØ,Øg
```

Les résultats sont les suivants :

a) en suspension dans le gel aqueux, les microsphères de 2 μm de diamètre pénètrent dans les différentes assises de la couche cornée ainsi qu'à l'intérieur des canaux folliculaires. Les microsphères de 5 μm sont rarement présentes dans la couche cornée, après une minute de massage, et se localisent plutôt à l'entrée des canaux folliculaires ; cette tendance à pénétrer les follicules est un peu plus prononcée après 5 minutes de massage. Les microsphères de 12 μm de diamètre ne pénètrent ni dans la couche cornée ni dans les canaux folliculaires.

b) le véhicule eau-silicone a une influence sur la pénétration des microsphères de 2 μm : celles-ci sont plus nombreuses à l'intérieur des follicules sébacés et présentent une répartition uniforme dans la couche cornée. Par contre, avec ce véhicule, on ne trouve pratiquement pas de microsphères de 5 μm dans la couche cornée : elles sont localisées très profondément dans les follicules au voisinage des glandes sébacées ; le massage a également, dans ce cas, une influence favorable sur la pénétration des microsphères dans le compartiment folliculaire. Comme dans le cas du gel aqueux, les microsphères de 12 μm de diamètre ne pénètrent ni dans la couche cornée, ni dans les follicules.

Les exemples 1 à 6 ci-après décrivent des procédés de fabrication de microsphères de poly-β-alanine fluorescentes ou chargées de produits actifs et ayant le diamètre désiré.

Exemple 1

Préparation de microsphères de poly-β-alanine fluorescentes

Etape A : Préparation des sphères de poly-β-alanine en suspension.

Dans un réacteur de 3 litres muni d'un agitateur de type à ancre d'un diamètre de 9Ø mm, d'une arrivée d'azote, d'une ampoule d'addition et d'une tête de colonne à distiller, on introduit : 1125g de toluène, 444g de tert-butanol et Ø,75g de copolymère (octadécène / anhydride maléïque) (vendu sous la dénomination commerciale "PA-18" par la Société "GULF"). Après chauffage de ce mélange à 7Ø°C, on ajoute 15Øg d'acrylamide. On porte alors la température à 1ØØ°C et on distille 9Øml du mélange azéotrope (eau/toluène/tert-butanol). Après la fin de la distillation, on refroidit le mélange réactionnel à 8Ø°C, et on ajuste la vitesse d'agitation à 6ØØt/min. On ajoute alors en 1Ø minutes une solution de 3,36g de tert-butylate de potassium dans 62 g de tert-butanol. L'ampoule d'addition est rincée par 75g de toluène. Après agitation pendant 5 heures à 8Ø°C, on laisse revenir à température ambiante. On ajoute ensuite, goutte à goutte, au mélange 11,25ml d'acide chlorhydrique concentré.

Etape B. Réticulation des sphères de poly-β-alanine.

A la suspension de microsphères de poly-β-alanine ainsi obtenue, on ajoute, en 3Ø minutes, sous une agitation à 6ØØt/min et à une température de 5Ø°C, 42g d'une solution aqueuse à 25 de glutaraldéhyde. Après avoir maintenu l'agitation pendant 4 heures à cette température, on laisse revenir la suspension à température ambiante.

Après décantation, les solvants surnageants sont éliminés et les microsphères sont lavées deux fois par 5ØØ ml d'éthanol. L'essorage après chaque lavage est effectué par centrifugation à 3.5ØØ t/min. Un lavage par 15 litres d'eau est ensuite effectué en continu, puis l'eau est éliminée jusqu'à un volume final de mélange de 6ØØ ml.

La poly-β-alanine réticulée est ensuite séchée par lyophilisation et l'on obtient 135g d'une poudre blanche, dont le diamètre des microsphères est en moyenne de 4,8Ø ± 1,1 μm, déterminé par la technique d'analyse d'images sur un appareil "QUANTIMET 52Ø" commercialisé par la Société "CAMBRIDGE INSTRUMENTS CO".

Etape C: Réaction avec le diamino-1,6 hexane

A une suspension de 2Øg des sphères de poly-β-alanine obtenues dans l'étape B dans 1ØØg d'eau, on ajoute 2Øg de diamino-1,6 hexane. On laisse sous agitation pendant 24 heures à la température ambiante, puis on essore sur verre fritté n° 4 ; enfin, on lave à l'eau jusqu'à ce que les eaux de lavage soient à un pH neutre.

Etape D : Fixation du produit fluorescent.

Les microsphères obtenues dans l'étape C sont mises en suspension dans 8Ø ml de solution tampon pH = 8,9 (27Ø ml de solution NaHCO3 Ø,1 M amenés à pH = 8,9 par addition d'environ 3Ø ml de solution de Na2 CO3 Ø,1 M). Dans cette suspension, on introduit 2g de chlorure de dansyle en solution dans 8Øg d'acétone. Le mélange est chauffé pendant 1Ø minutes au reflux du solvant, puis essoré sur verre fritté n° 4, enfin lavé à l'acétone jusqu'à disparition de toute trace de chlorure de dansyle dans le solvant de lavage contrôlé par détection U.V. à 25Ø nm. Les sphères sont d'abord séchées à l'air puis sous pression réduite à la température ambiante. La couleur finale des microsphères est jaune clair.

Example 2

Préparation de microsphères de poly-β-alanine chargées de péroxyde de benzoyle

Etape A : Préparation de sphères de poly-β-alanine en suspension.

Cette étape est identique à l'étape A de l'exemple 1.

Etape B : Réticulation des microsphères de poly-β-alanine

A une suspension de microsphères de poly-β-alanine obtenue à l'étape A, maintenue sous vive agitation (6ØØt/mn) et à une température de 5Ø°C, on ajoute régulièrement, en 15 minutes, 18g de solution aqueuse à 25 % en poids de glutaraldéhyde. Après avoir maintenu l'agitation pendant 4 heures à cette température, on laisse revenir la suspension à température ambiante. Après décantation, les solvants surnageants sont éliminés et les microsphères sont lavées deux fois par 5ØØ ml d'éthanol. L'essorage, après chaque lavage est effectué par centrifugation (35ØØt/min). Un lavage par 15 litres d'eau est ensuite effectué en continu puis l'eau est éliminée jusqu'à un volume final de mélange de 6ØØ ml. Le polymère gonflé est enfin séché par lyophilisation et l'on obtient 132g de poudre blanche, dont le diamètre des microsphères est en moyenne de 4,Ø5 ±2,Ø2 µm, mesuré selon la même méthode qu'à l'Etape B de l'exemple 1.

Etape C: Réduction des fonctions aldéhydes résiduelles.

A 15Øg de microsphères de poly-β-alanine réticulée obtenues à l'étape B, on ajoute 2,2 litres d'eau et on homogénéise par agitation. Après refroidissement à une température comprise entre 5 et 1Ø°C, on ajoute lentement une solution refroidie de borohydrure de sodium dans l'eau (5,2g de NaBH4 dans 6ØØml d'eau refroidie à 5°C). On maintient le milieu réactionnel entre 5 et 1Ø°C pendant 5 heures puis on amène le pH à 7 par addition d'acide acétique.

Après centrifugation du mélange et dispersion du résidu solide dans 45Ø ml d'eau, on le soumet à un lavage en continu par 5 litres d'eau (lavage en cellule "AMICON" équipée de filtre DIAPOR 0,2µm, pression 2 bars, agitation durant la totalité du lavage). Les microsphères hydratées sont ensuite séchées par lyophilisation. L'absence de coloration en présence du réactif de Schiff permet de conclure que les fonctions aldéhydes résiduelles ont été réduites. Après analyse, le diamètre des microsphères est identique à celui des microsphères de départ.

Etape D : Introduction du produit actif.

On dissout 44,5 g de peroxyde de benzoyle (à 75 % en poids) dans un mélange composé de 1125g d'acétone et de 375g d'eau ; puis 5Øg des microsphères préparées dans l'étape C sont mises en suspension dans cette solution. La suspension est concentrée à l'évaporateur rotatif, sous pression réduite, à une température ne dépassant pas 35°C, jusqu'à un poids total de 262g de suspension.

La teneur de la suspension obtenue en peroxyde de benzoyle est de 9,1 % en poids.

Exemple 3

Préparation de microsphères de poly-β-alanine chargées de nicotinate de benzyle.

Etapes A à C : Préparation des microsphères.

Les étapes A à C sont effectuées comme dans l'exemple 2.

Etape D : Introduction du produit actif.

On dissout 2 g de nicotinate de benzyle dans un mélange composé de 4∅g d'eau et de 4∅g d'éthanol ; puis 1∅g de microsphères préparées à l'étape C sont mis en suspension dans cette solution. La suspension est maintenue sous agitation pendant 2 heures, puis l'éthanol est éliminé à l'évaporateur rotatif, la température étant maintenue à une valeur inférieure à 35°C. Enfin, les microsphères sont séchées par lyophilisation.

Exemple 4

Préparation de microsphères de poly-β-alanine chargées de nicotinate de benzyle.

Les étapes A à C sont effectuées comme dans l'exemple 1 et l'étape D d'introduction de nicotinate de benzyle, comme produit actif, comme dans l'exemple 3.

Exemple 5

Préparation de microsphères de poly-β-alanine chargées d'acide rètinoïque.

Etapes A à C : Préparation des microsphères.

Les étapes A à C sont effectuées comme dans l'exemple 2.

Etape D : Introduction du produit actif.

On dissout 15 mg de butyl hydroxy toluène (antioxydant) dans 3∅g de propylène glycol-1,2, à une température de 3∅°C. A température ambiante, 24mg d'acide rétinoïque sont dissous dans 1∅g du mélange obtenu ci-dessus, sous argon et à l'abri de la lumière. La solution obtenue est filtrée à l'aide de filtres "Millipore" ∅,2 µm. 5g des microsphères préparées dans l'étape C sont mises en suspension dans cette solution à l'abri de la lumière et sous courant d'argon. Le mélange est réalisé à l'aide d'une spatule. Après deux heures d'absorption, une poudre jaune est obtenue. Le dosage de l'acide rétinoïque dans le spectrophotomètre ($\lambda = 358,8$ nm), après désorption du principe actif dans du diméthylsulfoxide.

Concentration théorique : ∅,16 %,
Concentration calculée : ∅,157 %

Exemple 6: Préparation de microsphères de poly-β- alanine chargées de chlorhydrate de clonidine

Etapes A à C : Préparation des microsphères

Les étapes A et C sont effectuées comme dans l'exemple 2.

Etape D : Introduction du produit actif.

On dissout 37,5 mg de chlorhydrate de clonidine dans 15 g d'eau à l'abri de la lumière, puis 3g de microsphères préparées à l'étape C sont ajoutées à 12g de la solution précédente. Le mélange est réalisé à l'aide d'une spatule. Après 2 heures d'absorption, on obtient une poudre blanche. Les microsphères sont alors séchées par lyophilisation. Le dosage du chlorhydrate de clonidine, dans le produit fini, est réalisé par analyse HPCL, après désorption du principe actif.
Concentration calculée : 1 %.

Exemple 7 : Préparation de microsphères de poly-β-alanine chargées de minoxidil.

Etapes A à C : Préparation des microsphères.

Les étapes A et C sont effectuées comme dans l'exemple 2.

Etape D : Introduction du produit actif.

Dans un mélange composé de 75g d'éthanol et 75g d'eau, 2g de Minoxidil sont dissous à 3Ø°C. Dans cette solution, on introduit 8g de sphères de poly-β-alanine obtenues suivant l'étape C. Le mélange est agité pendant 1 heure à l'évaporateur rotatif, puis on évapore le solvant jusqu'à l'obtention d'un gel. Le gel est congelé sous agitation, puis lyophilisé.

Concentration calculée : 11,6 % (par dosage UV à 23Ønm après mise en suspension dans de l'éthanol).

Exemple 8 : Préparation de microsphères de corps gras chargées d'acide rétinoïque.

Etape A : Préparation de la solution de principe actif.

2ØØmg d'acide all-trans rétinoïque sont dissous à l'abri de la lumière, dans 5 ml de dichloro-1,2-éthane.

Etape B : Enrobage du principe actif dans des microsphères de corps gras.

Dans un réacteur en acier inoxydable, muni d'une arrivée d'azote et équipé d'un agitateur magnétique et d'une plaque chauffante, on introduit : 4,75g de tristéarine et 25Ømg de monostéarate de glycérol. Le mélange est réalisé par agitation à la température de 8Ø°C. Puis on ajoute, en absence de lumière, la solution de principe actif préparée dans l'étape A. Le mélange obtenu est maintenu sous agitation à 8Ø°C puis insufflé, sous une pression d'azote de 7 bars, dans une buse de pulvérisation raccordée au réacteur Appareil "1/4 JCO-SS-SU.E15B-SS", EMANI). Les microsphères constituées par l'enrobage d'acide rétinoïque dans le mélange de corps gras tristéarine-monostéarate de glycérol, sont alors formées en aval de cette buse de pulvérisation à l'intérieur d'un caisson de filtration (longueur : 85cm), puis collectées sur une grille (Millipore, 24cm de diamètre, de préférence, "1 YY3Ø 293 58"). On obtient une poudre de couleur jaune. La teneur en acide rétinoïque des microsphères obtenues est de 2,78 % en poids. Le diamètre des microsphères, déterminé par analyse d'image (appareil MOP-VIDEOPLAN, Kontron), est de 4,43 ± 1,Ø8 μm.

Les exemples 9 à 18 ci-dessous, concernent la préparation de compositions cosmétiques ou pharmaceutiques à partir des microsphères chargées en produit actif préparées dans les exemples 2 à 8.

Exemple 9 : Préparation de microsphères de poly(lactide-co-glycolide) chargées en acide 6-[3-(1-adamantyl 4-méthoxyphényl)] 2- naphtoïque

0,5 g de poly(lactide-co-glycolide) vendu par la société "DUPONT" sous la dénomination commerciale de "MEDISORB 5050 DL" et 5 mg d'acide 6-[3-(1-adamantyl 4-méthoxyphényl)] 2-naphtoïque sont solubilisés dans 15 ml de chlorure de méthylène. La solution organique obtenue est émulsionnée sous agitation mécanique (2000 tr/min) dans 100 ml d'un gel aqueux contenant 0,3 g d'hydroxypropylcellulose vendu par la société "AQUALON" sous la dénomination commerciale de "KLUCEL HF". L'agitation mécanique est maintenue pendant 2 heures , ce qui permet l'évaporation progressive et totale du chlorure de méthylène.

Les microsphères obtenues sont récupérées, lavées trois fois avec de l'eau distillée et lyophylisées. La distribution de taille des microsphères obtenues par cette méthode est analysée au microscope. Le diamètre des sphères est compris entre 1 et 15 μm, avec une taille moyenne de 5 μm ; plus de 80 % des microsphères ont un diamètre compris entre 3 et 10 μm.

On contrôle l'encapsulation de la façon suivante :

1) l'observation des microsphères en microscopie optique (fluorescence) montre des sphères fluorescentes et l'absence de cristaux libres de principe actif.

2) l'observation en microscopie électronique confirme l'absence de cristaux à l'extérieur des sphères ainsi que l'absence de cristaux à la surface des sphères.

Pour évaluer le taux d'encapsulation du principe actif dans les microsphères, on extrait un échantillon des microsphères ci-dessus obtenues (100 mg) par du tétrahydrofuranne (5 ml) ; puis on filtre ; le filtrat est analysé par chromatographie liquide à haute performance : le taux d'encapsulation de l'acide 6- [3-(1-adamantyl 4-méthoxyphényl)] 2- naphtoïque est de 0,75 %.

Exemple 10 : Préparation de microsphères de poly(lactide-co-glycolide) chargées en acide rétinoïque

Des microsphères chargées en acide rétinoïque peuvent être obtenues avec le même procédé de préparation qu'à l'exemple 9 : les 5 mg d'acide 6-[3-(1-adamantyl 4-méthoxyphényl)] 2-naphtoïque sont alors remplacés par 2 mg d'acide rétinoïque.

Exemple 11 : Préparation de microsphères de triglycérides chargées en N-benzylphénylacétoxy-acétamide

Des microsphères sont préparées à partir de triglycérides, à savoir une huile de palme hydrogénée commercialisée sous le nom de "SOFTISAN 154" par la société "DYNAMIT NOBEL", par un procédé d'atomisation à l'aide d'un ensemble de pulvérisation sous pression.

Dans un réacteur en acier inoxydable thermostaté, le triglyceride et le principe actif, à savoir le N-benzyl phényl acétoxyacétamide à 15 % en poids par rapport au poids de triglycérides, sont fondus à 90°C sous atmosphère d'azote et à l'abri de la lumière. Le mélange fondu est poussé par de l'azote (pression de $0,5.10^2$ kPa) jusqu'à la buse à un certain débit et l'atomisation est réalisée au niveau de la buse sous pression d'azote (pression de $3 \times 10^2$ kPa).

La pulvérisation est réalisée dans une cuve en acier inoxydable étanche, possédant un gradient de température, d'environ -150°C en bas à 20°C en haut. Ce gradient est créé par introduction préalable d'azote liquide au fond de la cuve.

De façon générale, en fonction du type de buse choisi, la pression d'azote de pulvérisation et le débit du liquide conditionnent le diamètre moyen des sphères obtenues. Ainsi, plus le débit est faible, plus les gouttelettes en sortie de buse et, par conséquent, les microsphères au bas de la cuve sont petites. D'autre part, plus la pression de pulvérisation est forte, plus les sphères ont un diamètre faible et une distribution en taille homogène.

Dans cet exemple, des microsphères régulières sont obtenues sans cristaux libres de principe actif visibles au microscope. Le diamètre des sphères varie de 1 à 15 μm, avec un diamètre moyen inférieur à 10 μm. Le taux de principe actif incorporé, dosé par chromatographie en phase liquide à haute performance, a été évalué à 15 %.

Exemple 12

On prépare un gel en procédant au mélange des ingrédients suivants :

```
Suspension des microsphères préparée
suivant l'exemple 2 ................... 262 g
Eau q.s.p. ............................. 900 g
Acide polyacrylique réticulé vendu
sous la dénomination commerciale
"Carbopol 940" par la Société
Goodrich BF"......................... 3,6 g
Soude q.s.p. ......................... pH = 7
```

Cette préparation appliquée sur la peau, par massage jusqu'à pénétration complète, deux fois par jour pendant 30 jours, a d'excellentes propriétés antiacnéiques.

Exemple 13

On prépare un gel en procédant au mélange des ingrédients suivants :

```
Suspension des microsphères préparée
suivant l'exemple 2 ................... 262 g
Eau q.s.p. ........................... 445 g
```

Cette préparation appliquée sur la peau, par massage jusqu'à pénétration complète, deux fois par jour pendant 3Ø jours, a d'excellentes propriétés antiacnéïques.

Exemple 14

On prépare un gel en procédant au mélange des ingrédients suivants :

```
Suspension des microsphères préparée
suivant l'exemple 2 ................. 262 g
Acide polyacrylique réticulé vendu
sous la dénomination commerciale
"Carbopol 94Ø" par
la Société "Goodrich BF" ............. 22 g
Eau q.s.p. ......................... 4,4Kg
Soude q.s.p. ...................... pH = 7
```

Cette préparation appliquée sur la peau, par passage jusqu'à pénétration complète, deux fois par jour pendant 3Ø jours, a d'excellentes propriétés antiacnéïques.

Exemple 15

On prépare un gel en procédant au mélange des ingrédients suivants :

```
Microsphères préparées suivant
l'exemple 3 ......................      1 g
Acide polyacrylique réticulé vendu
sous la dénomination commerciale
"Carbopol 94Ø" par
la Société "Goodrich BF" ............ Ø,4 g
Eau q.s.p. ......................... 1ØØ g
Soude q.s.p. ...................... pH = 7
```

Cette préparation appliquée, par massage jusqu'à pénétration complète, deux fois par jour pendant 3Ø jours, sur certaines parties du corps, par exemple les seins, contribue à leur raffermissement.

Exemple 16

On prépare un gel en procédant au mélange des ingrédients suivants :

```
Microsphères préparées suivant
l'exemple 4 ........................  1,2 g
Acide polyacrylique réticulé vendu
sous la dénomination commerciale
"Carbopol 940" par
la Société "Goodrich BF"............  0,4 g
Eau q.s.p. .........................  100 g
Soude q.s.p. ......................  pH = 7
```

Cette préparation appliquée, par massage jusqu'à pénétration complète, deux fois par jour pendant 30 jours, sur certaines parties du corps, par exemple le cou, contribue à leur raffermissement.

Exemple 17

On prépare un gel en procédant au mélange des ingrédients suivants :

```
- Microsphères obtenues à
  l'Exemple 5 ......................  33 g
- Huile de silicone vendue par la
  Société "Prolabo" sous la référence
  "Rhodorsil RTV 70141" q.s.p. ......  100 g
```

Cette préparation appliquée, sur la peau, par massage jusqu'à pénétration complète, deux fois par jour pendant 30 jours, a d'excellentes propriétés anti-acnéiques.

Exemple 18

On prépare un gel en procédant au mélange des ingrédients suivants :

```
- Microphères obtenues à
  l'Exemple 5 ......................  33 g
- Huile de silicone vendue par la
  Société "Dow Corning" sous la
  référence "DC 344" q.s.p. ........  100 g
```

Cette préparation appliquée sur la peau, par massage jusqu'à pénétration complète, deux fois par jour pendant 30 jours, a d'excellentes propriétés anti-acnéiques.

Exemple 19

On prépare un gel en procédant au mélange des ingrédients suivants :

```
    - Microsphères obtenues à
      l'Exemple 6 ........................    30 g
    - Dérivés cellulosiques vendus sous
      la dénomination commerciale
      "Klucel" par la Société "Hercules".  1,5 g
    - Eau  q.s.p. ......................... 100 g
```

Cette préparation appliquée sur la peau, par massage jusqu'à pénétration complète, deux fois par jour pendant 2 à 3 semaines, a d'excellentes propriétés antihypertensives.

Exemple 20

On prépare un gel en procédant au mélange des ingrédients suivants :

```
    - Microsphères obtenues à
      l'Exemple 7 ..................... 17,24 g
    - Dérivés cellulosiques vendus sous
      la dénomination commerciale
      "Klucel" par la Société "Hercules"   1,66 g
    - Eau ........................... 16,22 g
    - Propylèneglycol  q.s.p. ........... 100 g.
```

On applique ce gel deux fois par jour sur un cuir chevelu ayant subi une chute des cheveux importante. Après 3 mois de traitement, à raison de 1 ml par application, on observe une amélioration significative.

Exemple 21

On prépare un gel en procédant au mélange des ingrédients suivants :

```
    - Microsphères obtenues à
      l'Exemple 8 ......................    1,8  g
    - Dérivés cellulosiques vendus sous
      la dénomination commerciale
      "Klucel" par la Société "Hercules"   1,47 g
    - Eau q.s.p. .........................   100 g.
```

Cette préparation appliquée sur la peau, par massage jusqu'à pénétration complète, deux fois par jour pendant 30 jours, a d'excellentes propriétés anti-acnéiques.

Exemple 22

On prépare un gel en procédant au mélange des ingrédients suivants :

```
- Microsphères préparées suivant
  l'exemple 9.......................    15    g
- Acide polyacrylique réticulé
  vendu   sous la dénomination


"CARBOPOL 940" par la société
"GOODRICH BF"..................    0,4   g
- Eau.............q.s.p...........    100    g
- Soude..........q.s.p..........  pH = 7
```

Cette préparation appliquée sur la peau, par massage jusqu'à pénétration complète, deux fois par jour pendant 30 jours, a d'excellentes propriétés anti-acnéiques.

Exemple 23

On prépare un gel en procédant au mélange des ingrédients suivants :

```
- Microsphères préparées suivant
  l'exemple 10....................    5    g
- Acide polyacrylique réticulé
  vendu sous la dénomination
  "CARBOPOL 940" par la société
  "GOODRICH BF"..................    0,4   g
- Eau...........q.s.p............    100    g
- Soude.........q.s.p............  ph = 7
```

Cette préparation appliquée sur la peau, par massage jusqu'à pénétration complète, deux fois par jour pendant 30 jours, a d'excellentes propriétés anti-acnéiques.

Exemple 24

On prépare un gel en procédant au mélange des ingrédients suivants :

```
- Microsphères préparées suivant
  l'exemple 11....................    20    g
- Acide polyacrylique réticulé
  vendu sous la dénomination
  "CARBOPOL 940" par la société
  "GOODRICH BF"..................    0,4   g
- Eau...........q.s.p..........    100    g
- Soude .........q.s.p..........  ph = 7
```

Cette préparation appliquée sur la peau, par massage jusqu'à pénétration complète, deux fois par jour pendant 30 jours, a d'excellentes propriétés anti-inflammatoires.

**Revendications**

**Revendications pour les Etats contractants suivants : AT, BE, CH, DE, FR, GB, IT, LI, LU, NL, SE**

1. Composition pharmaceutique ou cosmétique pour application topique, contenant, dans un véhicule approprié, des microsphères de polymères ou de corps gras de point de fusion supérieur à 50°C chargées d'au moins un produit actif, caractérisée par le fait qu'au moins 80% en poids des microsphères ont un diamètre compris entre 3 μm et 10 μm.

2. Composition selon la revendication 1, caractérisée par le fait que le polymère est choisi dans le groupe formé par les polymères à base de styrène, les polymères à base de β-alanine, les polymères dérivés de l'acide acrylique ou méthacrylique, les polyesters dérivés de l'acide lactique et/ou glycolique, les protéines réticulées, et les protéines coagulées par la chaleur.

3. Composition selon la revendication 2, caractérisée par le fait que le polymère est à base de poly-β-alanine.

4. Composition selon la revendication 2, caractérisée par le fait que le polymère est un polyester dérivé de l'acide lactique et/ou glycolique.

5. Composition selon la revendication 1, caractérisée par le fait que le corps gras est choisi dans le groupe formé par les alcools gras et les dérivés d'alcools et d'acides gras.

6. Composition selon la revendication 5, caractérisée par le fait que le corps gras a un point de fusion compris entre 50°C et 100°C.

7. Composition selon l'une des revendications 1 à 6, caractérisée par le fait que le produit actif est choisi dans le groupe formé par les agents de traitement de l'acné, les agents de raffermissement de la peau, les agents de traitement des cheveux, les antifongiques, les astringents, les antibiotiques, les antiviraux, les antihypertenseurs, les antiangineux, les vasodilatateurs, les agents de traitement des maladies cardiovasculaires, les agents anti-inflammatoires, les anti-allergènes, les antiprurigineux, les facteurs de croissance de nature peptidique ou protéinique, les neurostimulants, les agents antidépresseurs, les composés naturels utilisés en recherche neurobiologique, les anesthésiants et les stéroïdes hormonaux.

8. Composition selon la revendication 7, caractérisée par le fait que, comme agent de traitement de l'acné, elle contient la vitamine A, l'acide rétinoïque ou un de ses dérivés, ou le peroxyde de benzoyle.

9. Composition selon la revendication 7, caractérisée par le fait qu'elle contient, comme agent anti-chute ou de repousse des cheveux, le minoxidil et, comme agent antiséborrhéique, la S-carboxyméthylcystéine ou l'octopirox.

10. Composition selon la revendication 7, caractérisée par le fait que, comme antifongique, elle contient la nystatine ou l'éconazole.

11. Composition selon la revendication 7, caractérisée par le fait que, comme astringent, elle contient le chlorure d'aluminium.

12. Composition selon la revendication 7, caractérisée par le fait que, comme antibiotique, elle contient l'érythromycine ou la tétracycline.

13. Composition selon la revendication 7, caractérisée par le fait que, comme agent anti-viral, elle contient la vidarabine.

14. Composition selon ta revendication 7, caractérisée par le fait que, comme antihypertenseur, elle contient le chlorhydrate de clonidine.

15. Composition selon la revendication 7, caractérisée par le fait que, comme vasodilatateur, elle contient de la bradikynine.

**16.** Composition selon la revendication 7, caractérisée par le fait que, comme agent de traitement des maladies cardio-vasculaires, elle contient un peptide du groupe des tachykinines, en particulier la "substance P".

**17.** Composition selon la revendication 7, caractérisée par le fait que, comme agent anti-inflammatoire, elle contient l'aspirine ou l'hydrocortisone ou ses dérivés.

**18.** Composition selon la revendication 7, caractérisée par le fait que, comme antiallergène, elle contient un chromoglycate.

**19.** Composition selon la revendication 7, caractérisée par le fait que, comme antiprurigineux, elle contient un dérivé de la phenothiazine.

**20.** Composition selon la revendication 7, caractérisée par le fait que, comme facteur de croissance de nature peptidique, elle contient le facteur de croissance épidermique (EGF).

**21.** Composition selon la revendication 7, caractérisée par le fait que, comme neurostimulant, elle contient la caféine ou la théophylline.

**22.** Composition selon la revendication 7, caractérisée par le fait que, comme antidépresseur, elle contient un sel de lithium.

**23.** Composition selon la revendication 7, caractérisée par le fait que, comme composé naturel utilisé en recherche neurobiologique, elle contient la capsaïcine.

**24.** Composition selon la revendication 7, caractérisée par le fait que, comme anesthésiant, elle contient la lidocaïne ou la procaïne.

**25.** Composition selon la revendication 7, caractérisée par le fait que, comme agent-antiangineux, elle contient la nitroglycérine.

**26.** Composition selon l'une des revendications 1 à 25, caractérisée par le fait que le véhicule est un véhicule aqueux, lipophile ou non, ou une huile.

**27.** Composition selon l'une des revendications 1 à 26, caractérisée par le fait que le véhicule est sous forme de liquide, de gel, de crème, de pâte, de pommade ou de poudre sèche.

**28.** Composition selon l'une des revendications 26 ou 27, caractérisée par le fait que le véhicule est un véhicule aqueux non lipophile constitué par une solution hydroalcoolique.

**29.** Composition selon l'une des revendications 26 ou 27, caractérisée par le fait que le véhicule est un véhicule aqueux lipophile constitué par une solution aqueuse de silicones.

**30.** Composition selon l'une des revendications 26 à 29, caractérisée par le fait que le véhicule est un véhicule aqueux contenant un agent gélifiant.

**31.** Composition selon l'une des revendications 26 ou 27, caractérisée par le fait que le véhicule est une huile constituée par un triglycéride d'acides gras en C8-C1Ø ou un mélange de plusieurs de ces triglycérides, de la vaseline, de l'huile de vaseline ou de la lanoline.

**32.** Composition selon l'une des revendications 1 à 31, caractérisée par le fait qu'elle contient de 1 % à 4Ø % en poids de microsphères, dont au moins 8Ø % ont des diamètres compris entre 3 et 1Ø μm.

**33.** Composition selon l'une des revendications 1 à 32, caractérisée par le fait qu'elle contient de Ø,Ø5 % à 6Ø % en poids de produit actif.

**Revendications pour les Etats contractants suivants : ES, GR**

**1.** Composition cosmétique pour application topique, contenant, dans un véhicule approprié, des microsphères de polymères ou de corps gras de point de fusion supérieur à 50° C chargées d'au moins un produit actif, caractérisée par le fait qu'au moins 80 % en poids des microsphères ont un diamètre compris entre 3 μm et 10 μm.

2.  Composition selon la revendication 1, caractérisé par le fait que le polymère est choisi dans le groupe formé par les polymères à base de styrène, les polymères à base de β-alanine, les polymères dérivés de l'acide acrylique ou méthacrylique, les polyesters dérivés de l'acide lactique et/ou glycolique, les protéines réticulées, et les protéines coagulées par la chaleur.

3.  Composition selon la revendication 2, caractérisée par le fait que le polymère est à base de poly-β-alanine.

4.  Composition selon la revendication 2, caractérisée par le fait que la polymère est un polyester dérivé de l'acide lactique et/ou glycolique.

5.  Composition selon la revendication 1, caractérisé par le fait que le corps gras est choisi dans le groupe formé par les alcools gras et les dérivés d'alcools et d'acides gras.

6.  Composition selon la revendication 5, caractérisée par le fait que le corps gras a un point de fusion compris entre 50° C et 100° C.

7.  Composition selon l'une des revendications 1 à 6, caractérisé par le fait que le produit actif est choisi dans le groupe formé par les agents de traitement de l'acné, les agents de raffermissement de la peau, les agents de traitement des cheveux, les antifongiques, les astringents, les antibiotiques, les antiviraux, les vasodilatateurs, les agents anti-inflammatoires, les anti-allergènes, les antiprurigineux, les facteurs de croissance de nature peptidique ou protéinique, les neurostimulants, les anesthésiants et les stéroïdes hormonaux.

8.  Composition selon la revendication 7, caractérisée par le fait que, comme agent de traitement de l'acné, elle contient la vitamine A, l'acide rétinoïque ou un de ses dérivés, ou le peroxyde de benzoyle.

9.  Composition selon la revendication 7, caractérisée par le fait qu'elle contient, comme agent anti-chute ou de repousse des cheveux, le minoxidil et, comme agent antiséborrhéique, la S-carboxyméthylcystéine ou l'octopirox.

10. Composition selon la revendication 7, caractérisée par le fait que, comme antifongique, elle contient la nystatine ou l'éconazole.

11. Composition selon la revendication 7, caractérisée par le fait que, comme astrigent, elle contient le chlorure d'aluminium.

12. Composition selon la revendication 7, caractérisée par le fait que, comme antibiotique, elle contient l'érythromycine ou la tétracycline.

13. Composition selon la revendication 7, caractérisée par le fait que, comme agent anti-viral, elle contient la vidarabine.

14. Composition selon la revendication 7, caractérisée par le fait que, comme vasodilatateur, elle contient de la bradikynine.

15. Composition selon la revendication 7, caractérisée par le fait que, comme agent anti-inflammatoire, elle contient l'aspirine ou l'hydrocortisone ou ses dérivés.

16. Compositions selon la revendication 7, caractérisée par le fait que, comme antiallergène, elle contient un chromoglycate.

17. Composition selon la revendication 7, caractérisée par le fait que, comme antiprurigineux, elle contient un dérivé de la phénothiazine.

18. Composition selon la revendication 7, caractérisée par le fait que, comme facteur de croissance de nature peptidique, elle contient le facteur de croissance épidermique (EGF).

19. Composition selon la revendication 7, caractérisée par le fait que, comme neurostimulant, elle contient la caféine ou la théophylline.

20. Composition selon la revendication 7, caractérisée par le fait que, comme anesthésiant, elle contient le lidocaïne ou la procaïne.

21. Composition selon l'une des revendications 1 à 20, caractérisée par le fait que le véhicule est un véhicule aqueux, lipophile ou non, ou une huile.

22. Composition selon l'une des revendications 1 à 21, caractérisée par le fait que le véhicule est sous forme de liquide, de gel, de crème, de pâte, de pommade ou de poudre sèche.

23. Composition selon l'une des revendications 21 ou 22, caractérisée par le fait que le véhicule est un véhicule aqueux non lipophile constitué par une solution hydroalcoolique.

24. Composition selon l'une des revendications 21 ou 22, caractérisée par le fait que le véhicule est un véhicule aqueux lipophile constitué par une solution aqueuse de silicones.

25. Composition selon l'une des revendications 21 ou 22, caractérisée par le fait que le véhicule est un véhicule aqueux contenant un agent gélifiant.

26. Composition selon l'une des revendications 21 ou 22, caractérisée par le fait que le véhicule est une huile constituée par un triglycéride d'acides gras en $C_8$-$C_{10}$ ou un mélange de plusieurs de ces triglycérides, de la vaseline, de l'huile de vaseline ou de la lanoline.

27. Composition selon l'une des revendications 1 à 26, caractérisée par le fait qu'elle contient de 1 % à 40 % en poids de microsphères, dont au moins 80 % ont des diamètres compris entre 3 et 10 $\mu$m.

28. Composition selon l'une des revendications 1 à 27, caractérisée par le fait qu'elle contient de 0,05 % à 60 % en poids de produit actif.

**Patentansprüche**

**Patentansprüche für folgende Vertragsstaaten : AT, BE, CH, DE, FR, GB, IT, LI, LU, NL, SE**

1. Pharmazeutisches oder kosmetisches Mittel zur topischen Anwendung, enthaltend in einem geeigneten Träger Mikrosphären aus Polymeren oder Fettkörpern mit einem Schmelzpunkt oberhalb von 50°C, die mit wenigstens einem Wirkstoff beladen sind, dadurch gekennzeichnet, daß wenigstens 80 Gewichtsprozent der Mikrosphären einen Durchmesser zwischen 3$\mu$m und 10$\mu$m besitzen.

2. Mittel nach Anspruch 1, dadurch gekennzeichnet, daß das Polymer ausgewählt ist unter Polymeren auf Basis von Styrol, Polymeren auf Basis von $\beta$-Alanin, Polymeren, die von Acryl- oder Methacrylsäure abgeleitet sind, Polyestern, die von Milchsäure und/oder Glycolsäure abgeleitet sind, vernetzten Proteinen und durch Wärme koagulierten Proteinen.

3. Mittel nach Anspruch 2, dadurch gekennzeichnet, daß es sich um ein Polymer auf Basis von Poly-$\beta$-alanin handelt.

4. Mittel nach Anspruch 2, dadurch gekennzeichnet, daß das Polymer ein Polyester ist, der von Milchsäure und/oder Glycolsäure abgeleitet ist.

5. Mittel nach Anspruch 1, dadurch gekennzeichnet, daß der Fettkörper ausgewählt ist unter Fettalkoholen und Derivaten von Fettalkoholen und Fettsäuren.

6. Mittel nach Anspruch 5, dadurch gekennzeichnet, daß der Fettkörper einen Schmelzpunkt zwischen 50 und 100°C besitzt.

7. Mittel nach einem der Ansprüche 1 bis 6, dadurch gekennzeichnet, daß der Wirkstoff ausgewählt ist unter Mitteln zur Behandlung von Akne, Mitteln zur Stärkung der Haut, Mitteln zur Behandlung der Haare, antifungischen Mitteln, adstringierenden Mitteln, Antibiotika, antiviralen Mitteln, antihypertensiven Mitteln, antianginösen Mitteln, Vasodilatatoren, Mitteln zur Behandlung kardiovaskulärer Erkrankungen, antiinflamatorischen Mitteln, antiallergischen Mitteln, Antipruriginosa, Wachstumsfaktoren mit Peptid- oder Proteinnatur, Neurostimulantien, Antidepressiva, natürlichen, in der neurobiologischen Forschung verwendeten Verbindungen, Anästhetika und Steroidhormonen.

8. Mittel nach Anspruch 7, dadurch gekennzeichnet, daß es als Mittel zur Behandlung von Akne Vitamin A, Retinoesäure oder ein Derivat davon oder Benzoylperoxid enthält.

9. Mittel nach Anspruch 7, dadurch gekennzeichnet, daß es als Mittel gegen Haarausfall oder zum Nachwachsen der Haare Minoxidil und als Antiseborrhoemittel S-Carboxymethylcystein oder Octopirox enthält.

10. Mittel nach Anspruch 7, dadurch gekennzeichnet, daß es als antifungisches Mittel Nystatin oder Econazol enthält.

11. Mittel nach Anspruch 7, dadurch gekennzeichnet, daß es als adstringierendes Mittel Aluminiumchlorid enthält.

12. Mittel nach Anspruch 7, dadurch gekennzeichnet, daß es als Antibiotikum Erythromycin oder Tetracyclin enthält.

13. Mittel nach Anspruch 7, dadurch gekennzeichnet, daß es als antivirales Mittel Vidarabin enthält.

14. Mittel nach Anspruch 7, dadurch gekennzeichnet, daß es als antihypertensives Mittel Clonidinhydrochlorid enthält.

15. Mittel nach Anspruch 7, dadurch gekennzeichnet, daß es als Vasodilator Bradikynin enthält.

16. Mittel nach Anspruch 7, dadurch gekennzeichnet, daß es als Mittel zur Behandlung kardiovaskulärer Erkrankungen ein Peptid der Tachykiningruppe, insbesondere die "Substanz P", enthält.

17. Mittel nach Anspruch 7, dadurch gekennzeichnet, daß es als antiinflamatorisches Mittel Aspirin oder Hydrokortison oder dessen Derivate enthält.

18. Mittel nach Anspruch 7, dadurch gekennzeichnet, daß es als antiallergisches Mittel Cromoglycat enthält.

19. Mittel nach Anspruch 7, dadurch gekennzeichnet, daß es als Antipruritus-Mittel ein Phenothiazinderivat enthält.

20. Mittel nach Anspruch 7, dadurch gekennzeichnet, daß es als Wachstumsfaktor peptidischer Natur den Epidermiswachstumsfaktor (EGF) enthält.

21. Mittel nach Anspruch 7, dadurch gekennzeichnet, daß es als Neurostimulans Coffein oder Theophyllin enthält.

22. Mittel nach Anspruch 7, dadurch gekennzeichnet, daß es als antidepressives Mittel ein Lithiumsalz enthält.

23. Mittel nach Anspruch 7, dadurch gekennzeichnet, daß es als natürliche in der neurobiologischen Forschung verwendete Verbindung Capsaicin enthält.

24. Mittel nach Anspruch 7, dadurch gekennzeichnet, daß es als Anästhetikum Lidocain oder Procain enthält.

25. Mittel nach Anspruch 7, dadurch gekennzeichnet, daß es als antianginöses Mittel Nitroglycerin enthält.

26. Mittel nach einem der Ansprüche 1 bis 25, dadurch gekennzeichnet, daß der Träger ein wässriger, lipophiler oder nichtlipophiler Träger oder ein Öl ist.

27. Mittel nach einem der Ansprüche 1 bis 26, dadurch gekennzeichnet, daß der Träger in Form einer Flüssigkeit, eines Gels, einer Creme, einer Paste, einer Salbe oder eines Trockenpulvers vorliegt.

**28.** Mittel nach einem der Ansprüche 26 oder 27, dadurch gekennzeichnet, daß der Träger ein wässriger, nichtlipophiler Träger ist, der durch eine wässrig-alkoholische Lösung gebildet wird.

**29.** Mittel nach einem der Ansprüche 26 oder 27, dadurch gekennzeichnet, daß der Träger ein wässriger lipophiler Träger ist, der durch eine wässrige Lösung von Silikonen gebildet wird.

**30.** Mittel nach einem der Ansprüche 26 bis 29, dadurch gekennzeichnet, daß der Träger ein wässriger Träger ist, der ein gelbildendes Mittel enthält.

**31.** Mittel nach einem der Ansprüche 26 oder 27, dadurch gekennzeichnet, daß der Träger ein Öl ist aus einem Triglycerid von C8-C10-Fettsäuren oder einer Mischung von mehreren dieser Triglyceride, Vaseline, Vaselineöl oder Lanolin.

**32.** Mittel nach einem der Ansprüche 1 bis 31, dadurch gekennzeichnet, daß es 1 bis 40 Gewichtsprozent Mikrosphären enthält, von denen wenigstens 80% einen Durchmesser zwischen 3 und 10μm besitzen.

**33.** Mittel nach einem der Ansprüche 1 bis 32, dadurch gekennzeichnet, daß es 0,05 bis 60 Gewichtsprozent Wirkstoff enthält.

**Patentansprüche für folgende Vertragsstaaten : GR, ES**

**1.** Kosmetiches Mittel zur topischen Anwendung, enthaltend in einem geeigneten Träger Mikrosphären aus Polymeren oder Fettkörpern mit einem Schmelzpunkt oberhalb von 50°C, die mit wenigstens einem Wirkstoff beladen sind, dadurch gekennzeichnet, daß wenigstens 80 Gewichtsprozent der Mikrosphären einen Durchmesser zwischen 3 μm und 10 μm besitzen.

**2.** Mittel nach Anspruch 1, dadurch gekennzeichnet, daß das Polymer ausgewählt ist unter Polymeren auf Basis von Styrol, Polymeren auf Basis von β-Alanin, Polymeren, die von Acryl- oder Methacrylsäure abgeleitet sind, Polyestern, die von Milchsäure und/oder Glycolsäure abgeleitet sind, vernetzten Proteinen und durch Wärme koagulierten Proteinen.

**3.** Mittel nach Anspruch 2, dadurch gekennzeichnet, daß es sich um ein Polymer auf Basis von Poly-β-alanin handelt.

**4.** Mittel nach Anspruch 2, dadurch gekennzeichnet, daß das Polymer ein Polyester ist, der von Milchsäure und/oder Glycolsäure abgeleitet ist.

**5.** Mittel nach Anspruch 1, dadurch gekennzeichnet, daß der Fettkörper ausgewählt ist unter Fettalkoholen und Derivaten von Fettkoholen und Fettsäuren.

**6.** Mittel nach Anspruch 5, dadurch gekennzeichnet, daß der Fettkörper einen Schmelzpunkt zwischen 50 und 100°C besitzt.

**7.** Mittel nach einem der Ansprüche 1 bis 6, dadurch gekennzeichnet, daß der Wirkstoff ausgewählt ist unter Mitteln zur Behandlung von Akne, Mitteln zur Stärkung der Haut, Mitteln zur Behandlung der Haare, antifungischen Mitteln, adstringierenden Mitteln, Antibiotika, antiviralen Mitteln, Vasodilatatoren, antiinflammatorischen Mitteln, antiallergischen Mitteln, Antipruriginosa, Wachstumsfaktoren mit Peptid- oder Proteinnatur, Neurostimulantien, Anästhetika und Steroidhormonen.

**8.** Mittel nach Anspruch 7, dadurch gekennzeichnet, daß es als Mittel zur Behandlung von Akne Vitamin A, Retinoesäure oder ein Derivat davon oder Benzoylperoxid enthält.

**9.** Mittel nach Anspruch 7, dadurch gekennzeichnet, daß es als Mittel gegen Haarausfall oder zum Nachwachsen der Haare Minoxidil und als Antiseborrhoemittel S-Carboxymethylcystein oder Octopirox enthält.

**10.** Mittel nach Anspruch 7, dadurch gekennzeichnet, daß es als antifungisches Mittel Nystatin oder Econazol enthält.

**11.** Mittel nach Anspruch 7, dadurch gekennzeichnet, daß es als adstringierendes Mittel Aluminiumchlorid enthält.

12. Mittel nach Anspruch 7, dadurch gekennzeichnet, daß es als Antibiotikum Erythromycin oder Tetracyclin enthält.

13. Mittel nach Anspruch 7, dadurch gekennzeichnet, daß es als antivirales Mittel Vidarabin enthält.

14. Mittel nach Anspruch 7, dadurch gekennzeichnet, daß es als Vasodilator Bradikynin enthält.

15. Mittel nach Anspruch 7, dadurch gekennzeichnet, daß es als antiinflamatorisches Mittel Aspirin oder Hydrokortison oder dessen Derivate enthält.

16. Mittel nach Anspruch 7, dadurch gekennzeichnet, daß es als antiallegisches Mittel Cromoglycat enthält.

17. Mittel nach Anspruch 7, dadurch gekennzeichnet, daß es als Antipruritus-Mittel ein Phenothiazinderivat enthält.

18. Mittel nach Anspruch 7, dadurch gekennzeichnet, daß es als Wachstumfaktor peptidischer Natur den Epidermiswachstumsfaktor (EGF) enthält.

19. Mittel nach Anspruch 7, dadurch gekennzeichnet, daß es als Neurostimulans Coffein oder Theophyllin enthält.

20. Mittel nach Anspruch 7, dadurch gekennzeichnet, daß es als Anästhetikum Lidocain oder Procain enthält.

21. Mittel nach einem der Ansprüche 1 bis 20, dadurch gekennzeichnet, daß der Träger ein wässriger, lipophiler oder nichtlipophiler Träger oder ein Öl ist.

22. Mittel nach einem der Ansprüche 1 bis 21, dadurch gekennzeichnet, daß der Träger in Form einer Flüssigkeit, eines Gels, einer Creme, einer Paste, einer Salbe oder eines Trockenpulvers vorliegt.

23. Mittel nach einem der Ansprüche 21 oder 22, dadurch gekennzeichnet, daß der Träger ein wässriger, nichtlipophiler Träger ist, der durch eine wässrig-alkoholische Lösung gebildet wird.

24. Mittel nach einem der Ansprüche 21 oder 22, dadurch gekennzeichnet, daß der Träger ein wässriger lipophiler Träger ist, der durch eine wässrige Lösung von Silikonen gebildet wird.

25. Mittel nach einem der Ansprüche 21 oder 22, dadurch gekennzeichnet, daß der Träger ein wässriger Träger ist, der ein gebildendes Mittel enthält.

26. Mittel nach einem der Ansprüche 21 oder 22, dadurch gekennzeichnet, daß der Träger ein Öl ist aus einem Triglycerid von $C_8$-$C_{10}$-Fettsäuren oder einer Mischung von mehreren dieser Triglyceride, Vaseline, Vaselineöl oder Lanolin.

27. Mittel nach einem der Ansprüche 1 bis 26, dadurch gekennzeichnet, daß es 1 bis 40 Gewischstprozent Mikrosphären enthält, von denen wenigstens 80 % einen Durchmesser zwischen 3 und 10 µm besitzen.

28. Mittel nach einem der Ansprüche 1 bis 27, dadurch gekennzeichnet, daß es 0,05 bis 60 Gewichtsprozent Wirkstoff enthält.

**Claims**

**Claims for the following Contracting States : AT, BE, CH, DE, FR, GB, IT, LI, LU, NL, SE**

1. Pharmaceutical or cosmetic composition for topical application containing, in a suitable carrier, microspheres or polymers or of fatty substances with a melting point higher than 50°C filled with at least one active product, characterised in that at least 80 % by weight of the microspheres have a diameter of between 3 µm and 10 µm.

2. Composition according to Claim 1, characterised in that the polymer is chosen from the group consisting

23

of styrene-based polymers, β-alanine-based polymers, polymers derived from acrylic or methacrylic acid, polyesters derived from lactic and/or glycolic acid, crosslinked proteins and proteins coagulated by heat.

3.  Composition according to Claim 2, characterised in that the polymer is based on poly-β-alanine.

4.  Composition according to Claim 2, characterised in that the polymer is a polyester derived from lactic and/or glycolic acid.

5.  Composition according to Claim 1, characterised in that the fatty substance is chosen from the group consisting of fatty alcohols and derivatives of alcohols and of fatty acids.

6.  Composition according to Claim 5, characterised in that the fatty substance has a melting point of between 50°C and 100°C.

7.  Composition according to one of Claims 1 to 6, characterised in that the active product is chosen from the group consisting of agents for treating acne, skin-reinforcing agents, agents for treating hair, antifungals, astringents, antibiotics, antivirals, antihypertensives, antianginals, vasodilators, agents for treating cardiovascular disorders, antiinflammatory agents, antiallergens, antipruritics, growth factors of peptidic or proteinic nature, neurostimulants, antidepressant agents, natural compounds employed in neurobiological research, anaesthetics and hormone steroids.

8.  Composition according to Claim 7, characterised in that it contains vitamin A, retinoic acid or one of its derivatives, or benzoyl peroxide, as agent for treating acne.

9.  Composition according to Claim 7, characterised in that it contains minoxidil as antiloss or hair regrowth agent and S-carboxymethylcysteine or octopirox as antiseborrhoeic agent.

10. Composition according to Claim 7, characterised in that it contains nystatin or econazole as antifungal.

11. Composition according to Claim 7, characterised in that it contains aluminium chloride as astringent.

12. Composition according to Claim 7, characterised in that it contains erythromycin or tetracycline as antibiotic.

13. Composition according to Claim 7, characterised in that it contains vidarabine as antiviral agent.

14. Composition according to Claim 7, characterised in that it contains clonidine hydrochloride as antihypertensive.

15. Composition according to Claim 7, characterised in that it contains bradikynin as vasodilator.

16. Composition according to Claim 7, characterised in that it contains a peptide of the tachykinins group, in particular "substance P", as agent for treating cardiovascular disorders.

17. Composition according to Claim 7, characterised in that it contains aspirin or hydrocortisone or its derivatives as antiinflammatory agent.

18. Composition according to Claim 7, characterised in that it contains a chromoglycate as antiallergen.

19. Composition according to Claim 7, characterised in that it contains a phenothiazine derivative as antipruritic.

20. Composition according to Claim 7, characterised in that it contains the epidermic growth factor (EGF) as growth factor of peptidic nature.

21. Composition according to Claim 7, characterised in that it contains caffeine or theophylline as neurostimulant.

22. Composition according to Claim 7, characterised in that it contains a lithium salt as antidepressant.

23. Composition according to Claim 7, characterised in that it contains capsaicine as natural compound employed in neurobiological research.

24. Composition according to Claim 7, characterised in that it contains lidocaine or procaine as anaesthetic.

25. Composition according to Claim 7, characterised in that it contains nitroglycerine as antianginal agent.

26. Composition according to one of Claims 1 to 25, characterised in that the carrier is an aqueous carrier, lipophilic or otherwise, or an oil.

27. Composition according to one of Claims 1 to 26, characterised in that the carrier is in the form of liquid, of gel, of cream, of paste, of pomade or of dry powder.

28. Composition according to either of Claims 26 and 27, characterised in that the carrier is a nonlipophilic aqueous carrier consisting of a hydroalcoholic solution.

29. Composition according to either of Claims 26 and 27, characterised in that the carrier is a lipophilic aqueous carrier consisting of an aqueous solution of silicones.

30. Composition according to one of Claims 26 to 29, characterised in that the carrier is an aqueous carrier containing a gelling agent.

31. Composition according to either of Claims 26 and 27, characterised in that the carrier is an oil consisting of a triglyceride of $C_8$-$C_{10}$ fatty acids or a mixture of several of these triglycerides, of vaseline, of liquid paraffin or of lanolin.

32. Composition according to one of Claims 1 to 31, characterised in that it contains from 1 % to 40 % by weight of microspheres at least 80 % of which have diameters of between 3 and 10 $\mu$m.

33. Composition according to one of Claims 1 to 32, characterised in that it contains from 0.05 % to 60 % by weight of active product.

**Claims for the following Contracting States : ES, GR**

1. Cosmetic composition for topical application containing, in a suitable carrier, microspheres or polymers or of fatty substances with a melting point higher than 50°C filled with at least one active product, characterised in that at least 80 % by weight of the microspheres have a diameter of between 3 $\mu$m and 10 $\mu$m.

2. Composition according to Claim 1, characterised in that the polymer is chosen from the group consisting of styrene-based polymers, β-alanine-based polymers, polymers derived from acrylic or methacrylic acid, polyesters derived from lactic and/or glycolic acid, crosslinked proteins and proteins coagulated by heat.

3. Composition according to Claim 2, characterised in that the polymer is based on poly-β-alanine.

4. Composition according to Claim 2, characterised in that the polymer is a polyester derived from lactic and/or glycolic acid.

5. Composition according to Claim 1, characterised in that the fatty substance is chosen from the group consisting of fatty alcohols and derivatives of alcohols and of fatty acids.

6. Composition according to Claim 5, characterised in that the fatty substance has a melting point of between 50°C and 100°C.

7. Composition according to one of Claims 1 to 6, characterised in that the active product is chosen from the group consisting of agents for treating acne, skin-reinforcing agents, agents for treating hair, antifungals, astringents, antibiotics, antivirals, vasodilators, antiinflammatory agents, antiallergens, antipruritics, growth factors of peptidic or proteinic nature, neurostimulants, anaesthetics and hormone steroids.

8. Composition according to Claim 7, characterised in that it contains vitamin A, retinoic acid or one of its derivatives, or benzoyl peroxide, as agent for treating acne.

9. Composition according to Claims 7, characterised in that it contains minoxidil as antiloss or hair regrowth agent and S-casboxymethylcysteine or octopirox as antiseborrhoeic agent.

10. Composition according to Claim 7, characterised in that it contains nystatin or econazole as antifungal.

11. Composition according to Claim 7, characterised in that it contains aluminium chloride as astringent.

12. Composition according to Claim 7, characterised in that it contains erythromycin or tetracycline as antibiotic.

13. Composition according to Claim 7, characterised in that it contains vidarabine as antiviral agent.

14. Composition according to Claim 7, characterised in that it contains bradikynin as vasodilator.

15. Composition according to Claim 7, characterised in that it contains aspirin or hydrocortisone or its derivatives as antiinflammatory agent.

16. Composition according to Claim 7, characterised in that it contains a chromoglycate as antiallergen.

17. Composition according to Claim 7, characterised in that it contains a phenothiazine derivative as antipruritic.

18. Composition according to Claim 7, characterised in that it contains the epidermic growth factor (EGF) as growth factor of peptidic nature.

19. Composition according to Claim 7, characterised in that it contains caffeine or theophylline as neurostimulant.

20. Composition according to Claim 7, characterised in that it contains lidocaine or procaine as anaesthetic.

21. Composition according to one of Claims 1 to 20, characterised in that the carrier is an aqueous carrier, lipophilic or otherwise, or an oil.

22. Composition according to one of Claims 1 to 21, characterised in that the carrier is in the form of liquid, of gel, of cream, of paste, of pomade or of dry powder.

23. Composition according to either of Claims 21 and 22, characterised in that the carrier is a nonlipophilic aqueous carrier consisting of a hydroalcoholic solution.

24. Composition according to either of Claims 21 and 22, characterised in that the carrier is a lipophilic aqueous carrier consisting of an aqueous solution of silicones.

25. Composition according to either of Claims 21 and 22, characterised in that the carrier is an aqueous carrier containing a gelling agent.

26. Composition according to either of Claims 21 and 22, characterised in that the carrier is an oil consisting of a triglyceride of $C_8$-$C_{10}$ fatty acids or a mixture of several of these triglycerides, of vaseline, of liquid paraffin or of lanolin.

27. Composition according to one of Claims 1 to 26, characterised in that it contains from 1 % to 40 % by weight of microspheres at least 80 % of which have diameters of between 3 and 10 $\mu$m.

28. Composition according to one of Claims 1 to 27, characterised in that it contains from 0.05 % to 60 % by weight of active product.